# EUROPEAN PATENT SPECIFICATION

(11) **EP 1 876 981 B1**
(45) Date of publication and mention of the grant of the patent: **16.05.2012**
(21) Application number: 06738397.6
(22) Date of filing: 15.03.2006
(51) Int. Cl.: A61B 17/70

(54) **KITS FOR TREATMENT OF THE SPINAL COLUMN USING ELONGATE SUPPORT MEMBERS**
KITS ZUR BEHANDLUNG DER WIRBELSÄULE MIT VERLÄNGERTEN STÜTZELEMENTEN
KITS DE TRAITEMENT DE LA COLONNE VERTEBRALE AU MOYEN D'ELEMENTS-SUPPORTS DE FORME ALLONGEE

(30) Priority: 16.03.2005 US 81436
(43) Date of publication of application: 16.01.2008
(73) Proprietor: Warsaw Orthopedic, Inc., Warsaw, IN 46581 (US)
(72) Inventor: BRUMFIELD, David, L., Collierville, TN 38017 (US)
(74) Representative: Hutchinson, Glenn Stanley
(86) International application number: PCT/US2006/009332
(87) International publication number: WO 2006/101898

(56) References cited:
- WO-A-01/45576
- DE-A1- 10 326 517
- DE-U1- 9 308 770
- US-A- 5 879 351
- US-A- 5 899 901
- US-A1- 2004 215 191
- US-A1- 2005 033 295

## Description

### FIELD OF THE INVENTION

The present invention relates generally to treatment of the spinal column, and more particularly relates to surgical kits for treatment of the spinal column using elongate support members.

### BACKGROUND

Various types and configurations of spinal instrumentation and constructs are currently used in the treatment of the spinal column to provide stabilization and support across multiple vertebral levels. One or more elongate spinal rods are sometimes used to provide such stabilization and support, with the rods attached to the vertebrae by a number of anchor elements including, for example, bone screws, hooks and/or various types and configurations of connectors.

The devices and components used in association with the spinal instrumentation and constructs are typically formed of either stainless steel or titanium materials to accommodate different construct criteria and requirements. For example, deformity surgeons may prefer systems formed of stainless steel due to increased rod stiffness and *in situ* bending properties, while degenerative and trauma surgeons may prefer titanium systems due to better imaging qualities. Since stainless steel is not bio-compatible with titanium, two complete instrumentation sets (including rod and anchor elements) in stainless steel and titanium must be provided to hospitals or distributors to satisfy construct stiffness requirements, desired imaging qualities, and/or other system needs. As a result, the manufacturing and inventory costs associated with providing two complete instrumentation sets for use in association with various spinal constructs can be quite high.

The document US-A-2004/0215191 (Kitchen) describes a spinal curvature correction device that has a flexible tube having one or multiple lumens extending longitudinally through substantially the entire length of the flexible member. The lumens are a plurality of channels formed to receive multiple longitudinal members, or rods. Each rod is shaped to a desired curvature of a healthy spine or a curvature that will affect a correction of a diseased spine. The hollow, flexible tube is surgically placed along the axis of the spine and fixed. After fixation to the spinal elements is structurally stable, a plurality of curved semi-rigid rods is placed within the channels of the flexible tube. As additional rods are placed within the hollow flexible member, increased force is applied to the spine by the device, thereby moving the spine towards the desired curvature.

Thus, there is a general need in the industry to provide improved surgical kits for treatment of the spinal column using elongate support members. The present invention satisfies this need and provides other benefits and advantages in a novel and unobvious manner.

### SUMMARY

The present invention relates generally to instrumentation used in association with treatment of the spinal column, and more particularly relates to surgical kits for treatment of the spinal column using elongate support members. While the actual nature of the invention covered herein can only be determined with reference to the claims appended hereto, certain forms of the invention that are characteristic of the preferred embodiments disclosed herein are described briefly as follows.

In a first aspect of the present invention, there is provided a surgical kit for treatment of the spinal column, comprising:
at least one first elongate support element configured for placement across multiple levels of the spinal column and formed of a first material having a first modulus of elasticity, at least one second elongate support element configured for placement across multiple levels of the spinal column and formed of a second material having a second modulus of elasticity different from said first modulus of elasticity,
a plurality of fixation elements each configured to engage any of said elongate support elements to the spinal column, each fixation element being configured to engage only one of said elongate support elements,
whereby, in use, a surgeon may choose at least one of said first elongate support element or said second elongate support element to provide a spinal construct with a desired degree of stiffness,
characterised by said first and second elongate support elements being one-piece elongate support elements, and the plurality of fixation elements being formed of a third material that is biocompatible with each of said first and second materials.

In accordance with a second aspect of the present invention, there is provided a method of making an apparatus for treatment of the spinal column, comprising:
providing at least one first elongate support element configured for placement across multiple levels of the spinal column and formed of a first material having a first modulus of elasticity;
providing a plurality of fixation elements each configured to engage any of the elongate support elements to the spinal column, each fixation element being configured to engage only one of said elongate support elements;
providing at least one second elongate support element configured for placement across multiple levels of the spinal column and formed of a second material having a second modulus of elasticity different from the first modulus of elasticity; determining a desired degree of stiffness associated with a spinal construct assembly for use in the treatment of the spinal column;
selecting at least one of said first elongate support element or said second elongate support element having a modulus of elasticity corresponding to the desired degree of stiffness associated with the spinal construct assembly; and
selecting at least two of the fixation elements for anchoring the selected elongate support element to at least two vertebrae to form a spinal construct assembly exhibiting the desired degree of stiffness,
characterised in that the method further comprises the steps of:
   providing said first and second elongate support elements to be one-piece support elements and the plurality of fixation elements to be formed of a third material that is biocompatible with each of the first and second materials.

Preferred embodiments of the present invention are defined in the appended dependent claims.

It is one object of the present invention to provide improved surgical kits for treatment of the spinal column using elongate support members. Further objects, features, advantages, benefits, and aspects of the present invention will become apparent from the drawings and description contained herein.

### BRIEF DESCRIPTION OF THE DRAWINGS

FIG. 1 is a spinal construct for treatment of the spinal column according to one form of the present invention, as attached to the posterior region of the spinal column.
FIG. 2 is a lateral view, partially in cross section, of the spinal construct shown in FIG. 1.
FIG. 3 is a kit including components used in association with treatment of the spinal column according to one form of the present invention.

### DESCRIPTION OF THE PREFERRED EMBODIMENTS

For the purposes of promoting an understanding of the principles of the invention, reference will now be made to the embodiments illustrated in the drawings and specific language will be used to describe the same. It will nevertheless be understood that no limitation on the scope of the invention is hereby intended, and that alterations and further modifications in the illustrated devices, and further applications of the principles of the invention as illustrated herein are contemplated as would normally occur to one skilled in the art to which the invention relates.

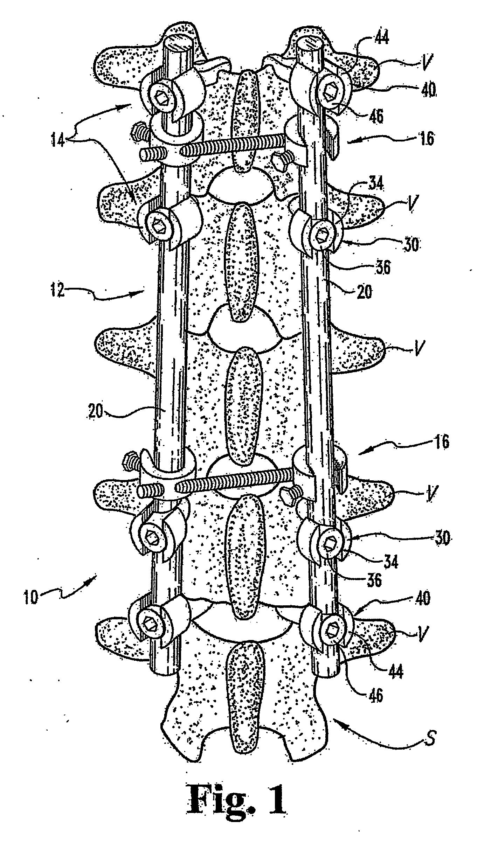

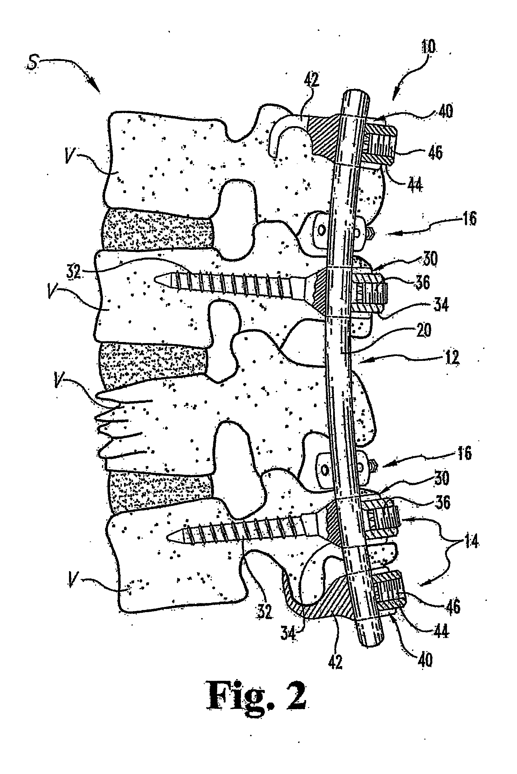

Referring to FIGS. 1 and 2, illustrated therein is spinal construct 10 for treatment of the spinal column according to one form of the present invention. The spinal construct 10 is engaged to the spinal column S and includes a number of elongate support elements 12 that extend across one or more vertebral levels, and a number of fixation elements 14 that anchor the elongate support elements 12 to the vertebrae V. Additionally, the spinal construct 10 may include a number of transverse connectors or link elements 16 that interconnect adjacent pairs of the elongate support elements 12. Further details regarding the spinal construct 10 will be discussed below.

In one embodiment of the invention, the elongate support elements 12 comprise spinal rods 20. However, it should be understood that other types of elongate support elements are also contemplated as falling within the scope of the present invention including, for example, plates, wires or other types of elongate support elements known to those of skill in the art. Additionally, although the spinal rods 20 are illustrated as having a generally circular outer cross section, it should be understood that other cross sectional shapes are also contemplated as falling within the scope of the invention including, for example, elliptical, hexagonal, rectangular or triangular cross sectional shapes, or any other suitable shape or configuration. Further, although the spinal construct 10 is illustrated as comprising a pair of elongate support members 12 positioned on opposite sides of the spinal column S, it should be understood that a single elongate support member 12 or three or more elongate support members 12 may also be used.

In a further embodiment of the invention, the fixation elements 14 comprise a number of bone screws 30 and a number of spinal hooks 40. However, it should be understood that other types and configurations of fixation elements are also contemplated including, for example, bolts, pins, staples, wires, connectors, cages or other types of fixation elements known to those of skill in the art. In the illustrated embodiment, the bone screws 30 each include a threaded shank portion 32 configured for engagement with vertebral bone, a U-shaped head portion 34 sized to receive one of the spinal rods 20 therein, and a set screw 36 threadedly engaged with the U-shaped head portion 34 to securely engage the spinal rod 20 to the bone screw 30. However, it should be understood that other types and configurations of bone screws and other techniques for engaging the spinal rod 20 to the bone screw are also contemplated as falling within the scope of the present invention. Additionally, in the illustrated embodiment, the spinal hooks 40 each include an anchoring blade portion 42 sized to wrap about a portion of a vertebra V, a U-shaped head portion 44 sized to receive one of the spinal rods 20 therein, and a set screw 46 threadedly engaged with the U-shaped head portion 44 to securely engage the spinal rod 20 to the spinal hook 40. However, it should be understood that other types and configurations of spinal hooks and other techniques for engaging the spinal rod 20 to the spinal hooks are also contemplated as falling within the scope of the present invention.

Further details regarding the structural configuration and operation of the bone screws 30 and the spinal hooks 40 are set forth in U.S. Patent No. 5,005,562 to Cotrel.

However, as mentioned above, it should be understood that other types and configurations of bone screws and spinal hooks are also contemplated as falling within the scope of the present invention. For example, U.S. Patent No. 5,527,314 to Brumfield et al. discloses a spinal implant system for correcting spinal deformities/abnormalities and including a pair of elongate spinal rods and a number of bone screws and hooks for engaging the spinal rods to the spinal column, the contents of which are also incorporated herein by reference.

In one embodiment of the invention, a first product inventory of spinal rods 20 is provided which are formed of a first material having a first modulus of elasticity to provide a select degree of stiffness. A second product inventory of spinal rods 20 is also provided which are formed of a second material having a second modulus of elasticity that is different from the first modulus of elasticity, thereby providing the first and second inventories of spinal rods 20 with different degrees of stiffness. In one embodiment of the invention, the ratio between the first and second moduli is at least about 1 to 1.05. In another embodiment, the ratio between the first and second moduli is at least about 1 to 1.25. In a further embodiment, the ratio between the first and second moduli is at least about 1 to 2. In one specific embodiment, the ratio between the first and second moduli is approximately 1 to 2.25. However, it should be understood that other ratios between the first and second moduli are also contemplated as falling within the scope of the present invention.

As should be appreciated, the surgeon may choose one or more spinal rods 20 from either of the first and second product inventories to provide the spinal construct 10 with a desired degree of stiffness to accommodate the specific criteria and requirements associated with treatment of the spinal column. Additionally, a third product inventory of fixation elements 14 is provided which are formed of a third material that is bio-compatible with each of the first and second materials associated with the first and second inventories of the spinal rods 20.

The first and second inventories of the spinal rods 20 and the third inventory of fixation elements 14 are each preferably formed of a titanium-compatible material. For definitional purposes, it should be understood that the term "titanium-compatible" encompasses any material that is bio-compatible with titanium, including titanium itself. At present, stainless steel is generally not considered to be a titanium-compatible material, and therefore is currently not contemplated for use in association with the manufacture of the spinal rods 20 or the fixation elements 14. However, it should be understood that future studies and developments may lead to a different conclusion regarding the biocompatibility between stainless steel and titanium-based materials, and that future use of stainless steel components in combination with titanium-based components is also contemplated as falling within the scope of the present invention.

In one specific embodiment of the invention, the first inventory of spinal rods 20 and the fixation elements 14 are each formed of Ti or Ti-CP (commercially pure titanium). In another specific embodiment, the second inventory of spinal rods 20 is formed of an alloy comprising Co and Cr, such as, for example, Cr-Co (chrome cobalt), CoCrMo (cobalt-chromium-molybdenum; also abbreviated as CCM or CoCr), or other Co-based or Cr-based alloy materials known to those of skill in the art. In a further specific embodiment, the second inventory of spinal rods 20 is formed of a titanium alloy such as Ti-6Al-4V (also abbreviated as Ti-6-4), a shape-memory alloy such as Nitinol (NiTi), or other titanium alloy materials known to those of skill in the art. In yet another specific embodiment, the first or second inventories of spinal rods 20 are formed of a plastic or polymeric material such as PEEK (polyetheretherketone), PLA (polylactate), or other plastic or polymeric materials known to those of skill in the art. Additionally, in other embodiments of the invention, auxiliary wiring may be used to secure one or more of the spinal rods 20 to the fixation elements 14. In such instances, the auxiliary wiring may be formed from MP35N, which is known to those of skill in the art to exhibit a high degree of strength and flexibility, and is also known to be compatible with both titanium and titanium-compatible materials.

It should be appreciated that spinal rods formed of titanium are more flexible compared to spinal rods formed of stainless steel. Specifically, titanium has a modulus of elasticity of about 103 Gpa, whereas stainless steel has a modulus of elasticity of about 200 Gpa. As should be apparent, titanium has a modulus of elasticity which is about one-half that of stainless steel. As a result, spinal constructs utilizing titanium rods provide a lower overall stiffness value compared to spinal constructs utilizing stainless steel rods. Additionally, it should also be appreciated that spinal rods or components formed of titanium provide better imaging qualities compared to spinal rods or components formed from stainless steel, which may be preferential to degenerative and trauma surgeons. Thus, in certain instances, a surgeon's use of spinal rods or components formed of titanium may be a preferred option for improved post-operative imaging qualities, even when a titanium-compatible rod of a greater modulus of elasticity is chosen for use in association with the spinal construct.

It should also be appreciated that spinal rods formed of a plastic or polymeric material (such as PEEK or PLA) are significantly more flexible compared to spinal rods formed of stainless steel. Specifically, PEEK has a modulus of elasticity of about 3.6 Gpa, whereas stainless steel has a modulus of elasticity of about 200 Gpa. As should be apparent, PEEK has a modulus of elasticity which is about 1.8% of the modulus of elasticity of stainless steel. As a result, spinal constructs utilizing PEEK rods provide a substantially lower overall stiffness value compared to spinal constructs utilizing stainless steel rods. Additionally, it should also be appreciated that spinal rods or components formed of PEEK provide better imaging qualities compared to spinal rods or components formed from stainless steel. Thus, in certain instances, a surgeon's use of spinal rods or components formed of PEEK may be a preferred option for improved post-operative imaging qualities.

It should further be appreciated that spinal rods formed of a titanium-compatible material (such as Co/Cr-based alloys or titanium alloys) may be selected to have a modulus of elasticity substantially similar to that of stainless steel. For example, Cr-Co has a modulus of elasticity of about 230 Gpa, whereas stainless steel has a modulus of elasticity of about 200 Gpa. As a result, spinal rods formed of Cr-Co provide similar bending properties and performance characteristics as compared to spinal rods formed of stainless steel, thereby providing the spinal construct assembly with an overall stiffness value similar to constructs made up of components formed entirely of stainless steel. Use of such spinal construct assemblies may be preferential to deformity surgeons due to increased rod stiffness and better *in situ* bending properties relative to spinal constructs utilizing rods formed of titanium. Additionally, rods formed from Cr-Co and other alloy materials provide improved fatigue performance and a lesser degree of notch sensitivity compared to rods formed from titanium.

As discussed above, in certain instances, a surgeon may prefer to use spinal rods having a degree of stiffness comparable to that of stainless steel. Additionally, the fixation elements 14 provided as part of the present invention are preferably formed exclusively of titanium or a titanium-compatible material. Further, at least at present, stainless steel is generally not considered to be a titanium-compatible material. Accordingly, as discussed above, the present invention provides a second inventory of spinal rods formed of a titanium-compatible material (e.g., a Co/Cr-based alloy or a titanium alloy). As a result, the surgeon can select a spinal rod having a degree of stiffness comparable to that of stainless steel, while at the same time being able to utilize a single inventory of fixation elements 14 formed exclusively of titanium or a titanium-compatible material.

As should now be apparent, the present invention addresses the disadvantages associated with maintaining separate inventories of stainless steel and titanium fixation elements by providing a product inventory of fixation elements (e.g., bone screw 30 and spinal hooks 40) formed of a single material such as titanium, and by providing two product inventories of elongate support elements 12 (e.g., spinal rods 20) formed of titanium and a titanium-compatible material (e.g., Co/Cr-based alloys, titanium alloys, plastics or polymers). By providing fixation elements 14 formed from a single material, manufacturing and inventory costs are reduced. Similarly, design and testing costs associated with bringing new fixation element designs to market are also reduced. Additionally, hospital inventory is reduced, yet the ability to provide spinal constructs that satisfy varying flexibility/stiffness requirements and desired imaging qualities is maintained. Indeed, a surgeon is given an intra-operative option to decide the most suitable construct mechanics (e.g., a more flexible construct via use of a titanium rod vs. a stiffer construct via use of titanium-compatible alloy materials having a higher modulus of elasticity, or a more rigid construct via use of rods formed of titanium or a titanium-compatible alloy material vs. a more flexible construct via use of a plastic or polymeric material having a lower modulus of elasticity).

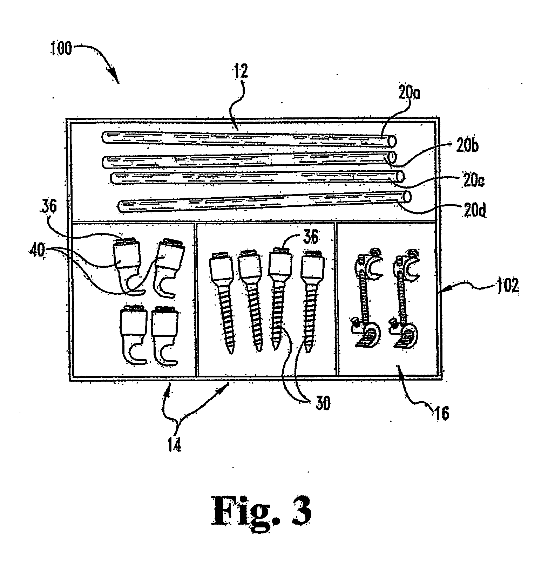

Referring to FIG. 3, shown therein is a surgical kit 100 including various components used to form a spinal construct for treatment of the spinal column according to one form of the present invention. In the illustrated embodiment of the invention, the surgical kit 100 includes the components used to form the spinal construct 10 illustrated in FIGS. 1 and 2 (e.g., elongate support elements 12, fixation elements 14, and transverse connectors or link elements 16). However, as discussed above, the use of other types and configurations of elongate support elements, fixation elements, and/or connector elements are also contemplated as falling within the scope of the present invention.

The illustrated embodiment of the surgical kit 100 comprises packaging 102 for containing and maintaining the spinal construct components in a sterilized condition prior to surgery. The components associated with the surgical kit 100 include a number of elongate support elements 12, a number of fixation elements 14, and a number of transverse connectors or link elements 16. Specifically, the surgical kit 100 includes a first pair of spinal rods 20a, 20b, a second pair of spinal rods 20c, 20d, a number of bone screws 30 (including setscrews 36), and a number of spinal hooks 40. However, as discussed above with regard to the spinal construct 10, the use of other types, configurations and quantities of elongate support elements and fixation elements to form other spinal constructs are also contemplated as falling within the scope of the present invention

Additionally, although not specifically illustrated in FIG. 3, it should be understood that the kit 100 may include one or more instruments or tools to aid in performing the designated surgical procedure including, for example, drivers, rod reducers and/or other types of instrumentation used in association with spinal surgery. Further information regarding surgical kits for use in association with treatment of the spine, including discussion on packaging and other components and instruments that may be included with the surgical kit 100, are set forth in U.S. Patent Application Serial No. 10/634,206 to Powers et al.

In one embodiment of the invention, the first pair of the spinal rods 20a, 20b is formed of a first material having a first modulus of elasticity to provide a select degree of stiffness, and the second pair of spinal rods 20c, 20d is formed of a second material having a second modulus of elasticity that is different from the first modulus of elasticity, thereby providing the first and second pairs of spinal rods with different degrees of stiffness. Accordingly, the surgeon may choose one or more spinal rods from either of the first and second pairs of spinal rods 20a, 20b and 20c, 20d to provide the spinal construct 10 with a desired degree of stiffness to accommodate the specific criteria and requirements associated with treatment of the spinal column. Additionally, the bone screws 30 and the spinal hooks 40 are formed of a third material that is bio-compatible with each of the first and second materials associated with the first and second pairs of spinal rods.

In one embodiment of the invention, each of the spinal rods 20a-20d, the bone screws 30 and the spinal hooks 40 are each formed of a titanium-compatible material. In a specific embodiment, the first pair of spinal rods 20a, 20b and the bone screws 30 and spinal hooks 40 are each formed of Ti or Ti-CP (commercially pure titanium). In another specific embodiment, the second pair of spinal rods 20c, 20d is formed of an alloy comprising Co and Cr, such as, for example, Cr-Co (chrome cobalt), CoCrMo (cobalt-chromium-molybdenum; also abbreviated as CCM or CoCr), or other Co-based or Cr-based alloy materials known to those of skill in the art. In a further embodiment, the second pair of the spinal rods 20c, 20d is formed of a titanium alloy such as Ti-6AI-4V (also abbreviated as Ti-6-4), a shape-memory alloy such as Nitinol (NiTi), or other titanium alloy materials known to those of skill in the art. In yet another specific embodiment, the first or second pairs of the spinal rods 20a, 20b and 20c, 20d may be formed of a plastic or polymeric material such as PEEK (polyetheretherketone), PLA (polylactate), or other plastic or polymeric materials known to those of skill in the art. Additionally, the surgical kit 100 may include a third pair of spinal rods formed from a plastic or polymeric material, with the third pair of spinal rods having a modulus of elasticity that is different from the modulus of elasticity associated with the first and second pairs of spinal rods 20a, 20b and 20c, 20d to provide the third pair of spinal rods with a different degree of stiffness compared to that of the first and second pairs of spinal rods.

As discussed above, spinal rods formed of titanium or plastic/polymeric materials are more flexible compared to spinal rods formed of stainless steel. As also discussed above, spinal rods formed of a titanium-compatible material such as Co/Cr-based alloys or titanium alloys may be selected to have a modulus of elasticity substantially similar to that of stainless steel. By providing the kit 100 with spinal rods having different degrees of stiffness, spinal constructs can be assembled which provide a desired degree of stiffness to satisfy the specific criteria and requirements associated with treatment of the spine. For example, a surgeon may select spinal rods from the kit 100 that have similar bending properties and performance characteristics as compared to spinal rods formed of stainless steel (e.g., rods formed of Cr-Co or titanium alloy materials). Additionally, the surgeon is also given the option to select spinal rods from the kit 100 that have a lower modulus of elasticity, and correspondingly a lower degree of stiffness, compared to spinal rods formed of stainless steel (e.g., rods formed of titanium or a plastic/polymer material such as PEEK).

As should now be apparent, the kit 100 addresses disadvantages associated with maintaining separate inventories of stainless steel and titanium fixation elements by providing a number of fixation elements formed of a single material (such as titanium) and by providing at least two groups of elongate support elements formed of titanium and/or titanium-compatible materials (e.g., Co/Cr-based alloys, titanium alloys, plastics or polymers). As a result, hospital inventory is reduced, yet the ability to provide spinal constructs that satisfy varying flexibility/stiffness requirements and desired imaging qualities is maintained.

For purposes of the present invention, the term "product inventory" includes groups of products or components that are made available to surgeons or other medical personal for the purpose of providing a spinal construct for use in treatment of the spine. As should be appreciated, the products or components that make up the surgical kit 100 (e.g., the elongate support elements 12, the anchor elements 14, and the transverse connectors elements 16) are considered to constitute product inventories.

While the invention has been illustrated and described in detail in the drawings and foregoing description, the same is to be considered as illustrative and not restrictive in character, it being understood that only the preferred embodiments have been shown and described and that all changes and modifications that come within the scope of the invention as defined by the claims are desired to be protected.

## Claims

1. A surgical kit for treatment of the spinal column, comprising:
at least one first elongate support element (20a, 20b) configured for placement across multiple levels of the spinal column and formed of a first material having a first modulus of elasticity,
at least one second elongate support element (20c, 20d) configured for placement across multiple levels of the spinal column and formed of a second material having a second modulus of elasticity different from said first modulus of elasticity,
a plurality of fixation elements (14) each configured to engage any of said elongate support elements (20a,20b,20c,20d) to the spinal column, each fixation element (14) being configured to engage only one of said elongate support elements (20a,20b,20c,20d),
whereby, in use, a surgeon may choose at least one of said first elongate support element (20a,20b) or said second elongate support element (20c,20d) to provide a spinal construct with a desired degree of stiffness,
**characterised by** said first and second elongate support elements being one-piece elongate support elements, and the plurality of fixation elements (14) being formed of a third material that is biocompatible with each of said first and second materials.

2. The surgical kit of claim 1, wherein a ratio between said first modulus of elasticity and said second modulus of elasticity is at least about 1 to 1.05.

3. The surgical kit of claim 2, wherein said ratio between said first modulus of elasticity and said second modulus of elasticity is at least about 1 to 1.25.

4. The surgical kit of claim 3, wherein said ratio between said first modulus of elasticity and said second modulus of elasticity is at least about 1 to 2.

5. The surgical kit of claim 1, wherein said first material is selected from the group consisting of titanium and a plastic material.

6. The surgical kit of claim 1, wherein said first material is commercially pure titanium.

7. The surgical kit of claim 5, wherein said first material or said second material is PEEK.

8. The surgical kit of claim 5, wherein said second modulus of elasticity of said second material is substantially similar to the modulus of elasticity of stainless steel.

9. The surgical kit of claim 5, wherein said second material is an alloy comprising Co and Cr.

10. The surgical kit of claim 5, wherein said second material is a titanium alloy.

11. The surgical kit of claim 1, wherein said first material is titanium; and wherein said second material is selected from the group consisting of Cr-Co, Co-Cr-Mo, Ti-6Al-4V and MP35N.

12. The surgical kit of claim 1, wherein said first material is titanium; and wherein said second material is a plastic material.

13. The surgical kit of claim 1, wherein said third material is selected from the group consisting of titanium and a titanium alloy.

14. The surgical kit of claim 1, wherein said elongate support elements comprise spinal rods.

15. The surgical kit of claim 1, wherein said fixation elements comprise bone screws.

16. The surgical kit of claim 15, wherein said fixation elements further comprise a number of connector elements for coupling said bone screws to said elongate support elements.

17. The surgical kit of claim 1, wherein said fixation elements comprise spinal hooks.

18. The surgical kit of claim 1, wherein said first and second materials are each titanium-compatible materials; and wherein said third material is selected from the group consisting of titanium, a titanium alloy, and an alloy comprising Co and Cr.

19. The surgical kit of claim 1, wherein said first and second materials are selected from the group consisting of titanium, a titanium alloy, MP35N, an alloy comprising Co and Cr, and PEEK.

20. A method of making an apparatus for treatment of the spinal column, comprising:
providing at least one first elongate support element (20a,20b) configured for placement across multiple levels of the spinal column and formed of a first material having a first modulus of elasticity;
providing a plurality of fixation elements (14) each configured to engage any of the elongate support elements (10a,20b,20c,20d) to the spinal column, each fixation element (14) being configured to engage only one of said elongate support elements (20a,20b,20c,20d);
providing at least one second elongate support element (20c,20d) configured for placement across multiple levels of the spinal column and formed of a second material having a second modulus of elasticity different from the first modulus of elasticity;
determining a desired degree of stiffness associated with a spinal construct assembly for use in the treatment of the spinal column;
selecting at least one of said first elongate support element (20a,20b) or said second elongate support element (20c,20d) having a modulus of elasticity corresponding to the desired degree of stiffness associated with the spinal construct assembly; and
selecting at least two of the fixation elements for anchoring the selected elongate support element (20a,20b,20c,20d) to at least two vertebrae to form a spinal construct assembly exhibiting the desired degree of stiffness,
**characterised in that** the method further comprises the steps of:
providing said first and second elongate support elements to be one-piece support elements and the plurality of fixation elements (14) to be formed of a third material that is biocompatible with each of the first and second materials.

21. The method of claim 20, wherein a ratio between the first modulus of elasticity and the second modulus of elasticity is at least about 1 to 1.05.

22. The method of claim 21, wherein the ratio between the first modulus of elasticity and the second modulus of elasticity is at least about 1 to 2.

23. The method of claim 20, wherein the first material is selected from the group consisting of titanium and a plastic material.

24. The method of claim 23, wherein the second material is an alloy comprising Co and Cr.

25. The method of claim 23, wherein the second material is a titanium alloy.

26. The method of claim 20, wherein the first material is titanium; and wherein the second material is selected from the group consisting of Cr-Co, Co-Cr-Mo, Ti-6Al-4V and MP35N.

27. The method of claim 20, wherein the first material is titanium; and wherein the second material is a plastic material.

28. The method of claim 20, wherein the third material is selected from the group consisting of titanium and a titanium alloy.

29. The method of claim 20 wherein said first material is titanium, said second material is an alloy comprising Co and Cr and said third material is titanium.

## Patentansprüche

1. Chirurgie-Kit zur Behandlung der Wirbelsäule, wobei der Kit folgendes umfasst:
mindestens ein erstes elongiertes Trägerelement (20a, 20b), das für eine Platzierung über eine mehreren Stufen der Wirbelsäule konfiguriert ist und aus einem ersten Material mit einem ersten Elastizitätsmodul ausgebildet ist;
mindestens ein zweites elongiertes Trägerelement (20c, 20d), das für eine Platzierung über mehrere Stufen der Wirbelsäule konfiguriert ist und aus einem zweiten Material mit einem zweiten Elastizitätsmodul ausgebildet ist, der sich von dem genannten ersten Elastizitätsmodul unterscheidet;
eine Mehrzahl von Fixierungselementen (14), die jeweils so konfiguriert sind, dass sie ein beliebiges der genannten Trägerelemente (20a, 20b, 20c, 20d) mit der Wirbelsäule in Eingriff bringen, wobei jedes der Fixierungselemente (14) so konfiguriert ist, dass es nur für eines der genannten elongierten Trägerelemente (20a, 20b, 20c, 20d) einen Eingriff herstellt;
wobei im Einsatz ein Chirurg zumindest das genannte erste elongierte Trägerelement (20a, 20b) oder das genannte zweite elongierte Trägerelement (20c, 20d) auswählen kann, um eine Wirbelsäulenkonstruktion mit einem gewünschten Steifheitsgrad bereitzustellen;
**dadurch gekennzeichnet, dass** es sich bei den genannten ersten und zweiten elongierten Trägerelementen um einteilige, elongierte Trägerelemente handelt, und wobei die Mehrzahl von Fixierungselementen (14) aus einem dritten Material besteht, das biologisch verträglich ist mit jedem der genannten ersten und zweiten Materialien.

2. Chirurgie-Kit nach Anspruch 1, wobei das Verhältnis zwischen dem genannten ersten Elastizitätsmodul und dem genannten zweiten Elastizitätsmodul mindestens etwa 1 zu 1,05 beträgt.

3. Chirurgie-Kit nach Anspruch 1, wobei das Verhältnis zwischen dem genannten ersten Elastizitätsmodul und dem genannten zweiten Elastizitätsmodul mindestens etwa 1 zu 1,25 beträgt.

4. Chirurgie-Kit nach Anspruch 1, wobei das Verhältnis zwischen dem genannten ersten Elastizitätsmodul und dem genannten zweiten Elastizitätsmodul mindestens etwa 1 zu 2 beträgt.

5. Chirurgie-Kit nach Anspruch 1, wobei das genannte erste Material aus der Gruppe ausgewählt wird, die Titan und einen Kunststoff umfasst.

6. Chirurgie-Kit nach Anspruch 1, wobei es sich bei dem genannten ersten Material um handelsübliches Reintitan handelt.

7. Chirurgie-Kit nach Anspruch 5, wobei es sich bei dem genannten ersten Material oder dem genannten zweiten Material um PEEK handelt.

8. Chirurgie-Kit nach Anspruch 5, wobei der genannte zweite Elastizitätsmodul des genannten zweiten Materials im Wesentlichen dem Elastizitätsmodul von Edelstahl entspricht.

9. Chirurgie-Kit nach Anspruch 5, wobei es sich bei dem genannten zweiten Material um eine Legierung handelt, die Co und Cr umfasst.

10. Chirurgie-Kit nach Anspruch 5, wobei es sich bei dem genannten zweiten Material um eine Titanlegierung handelt.

11. Chirurgie-Kit nach Anspruch 1, wobei es sich bei dem genannten ersten Material um Titan handelt, und wobei das genannte zweite Material aus der Gruppe ausgewählt wird, die Cr-Co, Co-Cr-Mo, Ti-6A1-4V und MP35N umfasst.

12. Chirurgie-Kit nach Anspruch 1, wobei es sich bei dem genannten ersten Material um Titan handelt, und wobei es sich bei dem genannten zweiten Material um einen Kunststoff handelt.

13. Chirurgie-Kit nach Anspruch 1, wobei das genannte dritte Material aus der Gruppe ausgewählt wird, die Titan und eine Titanlegierung umfasst.

14. Chirurgie-Kit nach Anspruch 1, wobei die genannten elongierten Trägerelemente Wirbelsäulenstäbe umfassen.

15. Chirurgie-Kit nach Anspruch 1, wobei die genannten Fixierungselemente Knochenschrauben umfassen.

16. Chirurgie-Kit nach Anspruch 15, wobei die genannten Fixierungselemente ferner eine Reihe von Verbindungselementen zur Kopplung der genannten Knochenschrauben mit den genannten elongierten Trägerelementen umfassen.

17. Chirurgie-Kit nach Anspruch 1, wobei die genannten Fixierungselemente Wirbelsäulenhaken umfassen.

18. Chirurgie-Kit nach Anspruch 1, wobei es sich bei den genannten ersten und zweiten Materialien jeweils um mit Titan verträgliche Materialien handelt; und wobei das genannte dritte Material aus der Gruppe ausgewählt wird, die Titan, eine Titanlegierung und eine Legierung umfasst, die Co und Cr umfasst.

19. Chirurgie-Kit nach Anspruch 1, wobei die genannten ersten und zweiten Materialien aus der Gruppe ausgewählt werden, die Titan, eine Titanlegierung, MP35N, eine Legierung, die Co und Cr umfasst, und PEEK umfasst.

20. Verfahren zur Herstellung einer Vorrichtung zur Behandlung der Wirbelsäule, wobei das Verfahren folgendes umfasst:
das Bereitstellen mindestens eines ersten elongierten Trägerelements (20a, 20b), das für eine Platzierung über mehrere Stufen der Wirbelsäule konfiguriert und aus einem ersten Material mit einem ersten Elastizitätsmodul ausgebildet ist;
das Bereitstellen einer Mehrzahl von Fixierungselementen (14), die jeweils für einen Eingriff jedes der elongierten Trägerelemente (20a, 20b, 20c, 20d) mit der Wirbelsäule konfiguriert sind, wobei jedes Fixierungselement (14) so konfiguriert ist, dass es einen Eingriff für nur eines der genannten elongierten Trägerelemente (20a, 20b, 20c, 20d) vorsieht;
das Bereitstellen mindestens eines zweiten elongierten Trägerelements (20c, 20d), das für eine Platzierung über mehrere Stufen der Wirbelsäule konfiguriert und aus einem zweiten Material ausgebildet ist, das einen zweiten Elastizitätsmodul aufweist, der sich von dem ersten Elastizitätsmodul unterscheidet;
das Bestimmen eines gewünschten Steifheitsgrads, der einer Wirbelsäulenkonstruktionseinheit zum Einsatz in der Behandlung der Wirbelsäule zugeordnet ist;
das Auswählen mindestens des genannten ersten elongierten Trägerelements (20a, 20b) oder des genannten zweiten eloongierten Trägerelements (20c, 20d) mit einem Elastizitätsmodul, der dem gewünschten Steifheitsgrad entspricht, der der Wirbelsäulenkonstruktionseinheit zugeordnet ist; und
das auswählen von mindestens zwei der Fixierungselemente für die Verankerung des ausgewählten elongierten Trägerelements (20a, 20b, 20c, 20d) an mindestens zwei Rückenwirbeln, so dass eine Wirbelsäulenkonstruktionseinheit gebildet wird, welche den gewünschten Steifheitsgrad aufweist;
**dadurch gekennzeichnet, dass** das Verfahren ferner die folgenden Schritte umfasst:
das Bereitstellen der genannten ersten und zweiten elongierten Trägerelemente als einteilige Trägerelemente, und das Ausbilden der Mehrzahl von Fixierungselementen (14) aus einem dritten Material, das mit jedem der genannten ersten und zweiten Materialien biologisch verträglich ist.

21. Verfahren nach Anspruch 20, wobei das Verhältnis zwischen dem ersten Elastizitätsmodul und dem zweiten Elastizitätsmodul mindestens etwa 1 zu 1,05 beträgt.

22. Verfahren nach Anspruch 21, wobei das Verhältnis zwischen dem genannten ersten Elastizitätsmodul und dem genannten zweiten Elastizitätsmodul mindestens etwa 1 zu 2 beträgt.

23. Verfahren nach Anspruch 20, wobei das ersten Material aus der Gruppe ausgewählt wird, die Titan und einen Kunststoff umfasst.

24. Verfahren nach Anspruch 23, wobei es sich bei dem zweiten Material um eine Legierung handelt, die Co und Cr umfasst.

25. Verfahren nach Anspruch 23, wobei es sich bei dem zweiten Material um eine Titanlegierung handelt.

26. Verfahren nach Anspruch 20, wobei es sich bei dem ersten Material um Titan handelt, und wobei das zweite Material aus der Gruppe ausgewählt wird, die Cr-Co, Co-Cr-Mo, Ti-6A1-4V und MP35N umfasst.

27. Verfahren nach Anspruch 20, wobei es sich bei dem ersten Material um Titan handelt; und wobei es sich bei dem zweiten Material um einen Kunststoff handelt.

28. Verfahren nach Anspruch 20, wobei das dritte Material aus der Gruppe ausgewählt wird, die Titan und eine Titanlegierung umfasst.

29. Verfahren nach Anspruch 20, wobei es sich bei dem genannten ersten Material um Titan handelt, wobei es sich bei dem genannten zweiten Material um eine Legierung handelt, die Co und Cr umfasst, und wobei es sich bei dem genannten dritten Material um Titan handelt.

## Revendications

1. Kit chirurgical pour le traitement de la colonne vertébrale, comprenant :
au moins un premier élément de support allongé (20a, 20b) configuré à des fins de placement entre de multiples niveaux de la colonne vertébrale et formé d'un premier matériau ayant un premier module d'élasticité,
au moins un second élément de support allongé (20c, 20d) configuré à des fins de placement entre de multiples niveaux de la colonne vertébrale et formé d'un deuxième matériau ayant un second module d'élasticité différent dudit premier module d'élasticité,
une pluralité d'éléments de fixation (14), chacun étant configuré pour venir en prise avec l'un quelconque desdits éléments de support allongés (20a, 20b, 20c, 20d) sur la colonne vertébrale, chaque élément de fixation (14) étant configuré pour venir en prise avec un seul desdits éléments de support allongés (20a, 20b, 20c, 20d),
moyennant quoi, en utilisation, un chirurgien peut choisir au moins l'un dudit premier élément de support allongé (20a, 20b) ou dudit second élément de support allongé (20c, 20d) pour fournir une structure vertébrale avec un degré voulu de rigidité,
**caractérisé en ce que** lesdits premier et second éléments de support allongés sont des éléments de support allongés monoblocs, et la pluralité d'éléments de fixation (14) sont formés d'un troisième matériau qui est biocompatible avec chacun desdits premier et deuxième matériaux.

2. Kit chirurgical selon la revendication 1, dans lequel un rapport entre ledit premier module d'élasticité et ledit second module d'élasticité est environ d'au moins 1 à 1,05.

3. Kit chirurgical selon la revendication 2, dans lequel ledit rapport entre ledit premier module d'élasticité et ledit second module d'élasticité est environ d'au moins 1 à 1,25.

4. Kit chirurgical selon la revendication 3, dans lequel ledit rapport entre ledit premier module d'élasticité et ledit second module d'élasticité est environ d'au moins 1 à 2.

5. Kit chirurgical selon la revendication 1, dans lequel ledit premier matériau est choisi dans le groupe constitué de titane et d'une matière plastique.

6. Kit chirurgical selon la revendication 1, dans lequel ledit premier matériau est du titane pur de qualité commerciale.

7. Kit chirurgical selon la revendication 5, dans lequel ledit premier matériau ou ledit deuxième matériau est du PEEK.

8. Kit chirurgical selon la revendication 5, dans lequel ledit second module d'élasticité dudit deuxième matériau est sensiblement similaire au module d'élasticité de l'acier inoxydable.

9. Kit chirurgical selon la revendication 5, dans lequel ledit deuxième matériau est un alliage comprenant du Co et du Cr.

10. Kit chirurgical selon la revendication 5, dans lequel ledit deuxième matériau est un alliage de titane.

11. Kit chirurgical selon la revendication 1, dans lequel ledit premier matériau est du titane ; et dans lequel ledit deuxième matériau est choisi dans le groupe constitué de Cr-Co, de Co-Cr-Mo, de Ti-6A1-4V et de MP35N.

12. Kit chirurgical selon la revendication 1, dans lequel ledit premier matériau est du titane ; et dans lequel ledit deuxième matériau est une matière plastique.

13. Kit chirurgical selon la revendication 1, dans lequel ledit troisième matériau est choisi dans le groupe constitué de titane et d'un alliage de titane.

14. Kit chirurgical selon la revendication 1, dans lequel lesdits éléments de support allongés comprennent des tiges vertébrales.

15. Kit chirurgical selon la revendication 1, dans lequel lesdits éléments de fixation comprennent des vis à os.

16. Kit chirurgical selon la revendication 15, dans lequel lesdits éléments de fixation comprennent en outre un certain nombre d'éléments de connexion pour coupler lesdites vis à os auxdits éléments de support allongés.

17. Kit chirurgical selon la revendication 1, dans lequel lesdits éléments de fixation comprennent des crochets vertébraux.

18. Kit chirurgical selon la revendication 1, dans lequel lesdits premier et deuxième matériaux sont chacun des matériaux compatibles avec le titane ; et dans lequel ledit troisième matériau est choisi dans le groupe constitué de titane, d'un alliage de titane et d'un alliage comprenant du Co et du Cr.

19. Kit chirurgical selon la revendication 1, dans lequel lesdits premier et deuxième matériaux sont choisis dans le groupe constitué de titane, d'un alliage de titane, de MP35N, d'un alliage comprenant du Co et du Cr, et de PEEK.

20. Procédé de fabrication d'un appareil pour le traitement de la colonne vertébrale, comprenant les étapes consistant à :
fournir au moins un premier élément de support allongé (20a, 20b) configuré à des fins de placement entre de multiples niveaux de la colonne vertébrale et formé d'un premier matériau ayant un premier module d'élasticité,
fournir une pluralité d'éléments de fixation (14), chacun étant configuré pour venir en prise avec l'un quelconque des éléments de support allongés (20a, 20b, 20c, 20d) sur la colonne vertébrale, chaque élément de fixation (14) étant configuré pour venir en prise avec un seul desdits éléments de support allongés (20a, 20b, 20c, 20d) ;
fournir au moins un second élément de support allongé (20c, 20d) configuré à des fins de placement entre de multiples niveaux de la colonne vertébrale et formé d'un deuxième matériau ayant un second module d'élasticité différent dudit premier module d'élasticité ;
déterminer un degré voulu de rigidité associé à un ensemble formant structure vertébrale pour une utilisation dans le traitement de la colonne vertébrale ;
sélectionner au moins l'un dudit premier élément de support allongé (20a, 20b) ou dudit second élément de support allongé (20c, 20d) ayant un module d'élasticité correspondant au degré voulu de rigidité associée à l'ensemble formant structure vertébrale ; et
sélectionner au moins deux des éléments de fixation pour l'ancrage de l'élément de support allongé sélectionné (20a, 20b, 20c, 20d) sur au moins deux vertèbres pour former un ensemble formant structure vertébrale présentant le degré voulu de rigidité,
**caractérisé en ce que** le procédé comprend en outre l'étape consistant à :
fournir lesdits premier et second éléments de support allongés comme des éléments de support allongés monoblocs et la pluralité d'éléments de fixation (14) étant formés d'un troisième matériau qui est biocompatible avec chacun desdits premier et deuxième matériaux.

21. Procédé selon la revendication 20, dans lequel un rapport entre le premier module d'élasticité et le second module d'élasticité est environ d'au moins 1 à 1,05.

22. Procédé selon la revendication 21, dans lequel un rapport entre le premier module d'élasticité et le second module d'élasticité est environ d'au moins 1 à 2.

23. Procédé selon la revendication 20, dans lequel le premier matériau est choisi dans le groupe constitué de titane et d'une matière plastique.

24. Procédé selon la revendication 23, dans lequel ledit deuxième matériau est un alliage comprenant du Co et du Cr.

25. Procédé selon la revendication 23, dans lequel ledit deuxième matériau est un alliage de titane.

26. Procédé selon la revendication 20, dans lequel le premier matériau est du titane ; et dans lequel ledit deuxième matériau est choisi dans le groupe constitué de Cr-Co, de Co-Cr-Mo, de Ti-6A1-4V et de MP35N.

27. Procédé selon la revendication 20, dans lequel le premier matériau est du titane ; et dans lequel ledit deuxième matériau est une matière plastique.

28. Procédé selon la revendication 20, dans lequel le troisième matériau est choisi dans le groupe constitué de titane et d'un alliage de titane.

29. Procédé selon la revendication 20, dans lequel ledit premier matériau est du titane, ledit deuxième matériau est un alliage comprenant du Co et du Cr et ledit troisième matériau est du titane.
